# EUROPEAN PATENT SPECIFICATION

(11) **EP 3 377 903 B1**
(45) Date of publication and mention of the grant of the patent: **21.08.2019**
(21) Application number: 16797460.9
(22) Date of filing: 17.11.2016
(51) Int. Cl.: G01N 33/574

(54) **USE OF CIRCULATING SERUM TROP-2 AS NEW TUMOR BIOMARKER**
VERWENDUNG VON ZIRKULIERENDEM SERUM TROP-2 ALS NEUER TUMORBIOMARKER
UTILISATION DE LA TROP-2 EN CIRCULATION DANS DU SÉRUM COMME NOUVEAU BIOMARQUEUR DE TUMEUR

(30) Priority: 19.11.2015 IT UB20155701
(43) Date of publication of application: 26.09.2018
(73) Proprietor: MEDITERRANEA THERANOSTIC S.R.L., 66034 Lanciano (CH) (IT)
(72) Inventor: GUERRA, Emanuela, 65012 Cepagatti (PE) (IT); TREROTOLA, Marco, 65127 Pescara (PE) (IT); ALBERTI, Saverio, 66034 Lanciano (CH) (IT)
(74) Representative: Primiceri, Maria Vittoria
(86) International application number: PCT/EP2016/025148
(87) International publication number: WO 2017/084763

(56) References cited:
- US-A1- 2007 099 251
- US-B2- 7 666 583
- THOMAS M. CARDILLO ET AL: "Sacituzumab Govitecan (IMMU-132), an Anti-Trop-2/SN-38 Antibody-Drug Conjugate: Characterization and Efficacy in Pancreatic, Gastric, and Other Cancers", BIOCONJUGATE CHEMISTRY., vol. 26, no. 5, 20 May 2015 (2015-05-20), pages 919-931, XP055268276, US ISSN: 1043-1802, DOI: 10.1021/acs.bioconjchem.5b00223
- Alexander N Starodub ET AL: "First-in-Human Trial of a Novel Anti-Trop-2 Antibody-SN-38 conjugate, Sacituzumab Govitecan, for the Treatment of Diverse Metastatic Solid Tumors Supplementary Information", , 4 June 2015 (2015-06-04), XP055268280, Retrieved from the Internet: URL:https://clincancerres.aacrjournals.org /content/suppl/2015/06/05/1078-0432.CCR-14 -3321.DC1/142817_2_supp_0_npfrhk.docx [retrieved on 2016-04-25]
- DAVID M GOLDENBERG ET AL: "Trop-2 is a novel target for solid cancer therapy with sacituzumab govitecan (IMMU-132), an antibody-drug conjugate (ADC)* Correspondence to", THE AACR-NCI-EORTC INTERNATIONAL CONFERENCE ON MOLECULAR TARGETS AND CANCER THERAPY, vol. 6, no. 26, 18 June 2015 (2015-06-18), pages 22496-22512, XP055269616,
- KAZUE NAKASHIMA ET AL: "Serological identification of TROP2 by recombinant cDNA expression cloning using sera of patients with esophageal squamous cell carcinoma", INTERNATIONAL JOURNAL OF CANCER, vol. 112, no. 6, 20 December 2004 (2004-12-20), pages 1029-1035, XP055057581, ISSN: 0020-7136, DOI: 10.1002/ijc.20517
- YICHAO XIA ET AL: "Expression of tumor-associated calcium signal transducerï 2 in patients with salivary adenoid cystic carcinoma: Correlation with clinicopathological features and prognosis", ONCOLOGY LETTERS, 31 July 2014 (2014-07-31), XP055269870, GR ISSN: 1792-1074, DOI: 10.3892/ol.2014.2400

## Description

### Field of the invention

The present invention relates to the use of circulating serum Trop-2 as a biomarker that can be used in the clinic as an indicator of tumor burden and to monitor the progression of tumor disease and the efficacy of anti-tumor therapies. The present invention also relates to methods to detect the Trop-2 protein in unfractionated serum.

### Background of the invention

Proteins associated with the initiation and metastasis of tumors have been analyzed and used in clinical practice for more than 30 years. These molecules have been defined as "tumor markers" or "biomarkers". Such biomarkers can be measured both in the tumor tissue and in the body fluids. Biomarkers that are released by the tumor into the bloodstream (i.e. serum biomarkers), can be used for the non-invasive discovery of tumor masses that are below the detection limits, for the evaluation of tumor spreading at different stages of disease and for monitoring tumor evolution and tumor relapse following therapy. This is even more important for tumors with high prevalence, for example breast, colorectal, stomach, pancreas, prostate, uterus, endometrium, ovary, lung, bladder, kidney and thyroid tumors.

Breast cancer and colorectal cancer are extremely relevant causes of tumor death in Western countries. According to recent statistics (Siegel, Miller et al. 2015), colorectal cancer accounts for about 8% of new cases of tumor in 2015, while breast cancer accounts for more than 14%.

Many factors are associated with the onset of breast cancer (Ekbom, Trichopoulos et al. 1992, Colditz, Willett et al. 1993, Easton, Bishop et al. 1993, Morrow 1994, Madigan, Ziegler et al. 1995, Newcomb, Storer et al. 1996, Parker, Tong et al. 1996), but despite the fact that the incidence of this cancer has increased over the last 30-40 years, the mortality rate has stabilized. Probably this is due to both a more accurate early diagnosis and an improvement in the selection of therapies (Tabar, Fagerberg et al. 1985, de Koning, Boer et al. 1995). These therapies include surgical removal, radiotherapy, chemotherapy, hormonal therapy and immunotherapy. The therapy of localized breast cancer, eg. surgical excision and/or radiotherapy, has curative intent.

In recent years, the prognosis for patients with early stage breast cancer improved, mainly due to hormonal therapy and chemotherapy (Bonadonna, Brusamolino et al. 1976, Bonadonna, Valagussa et al. 1995). At present in developed countries more than 70% of patients with breast cancer survive for more than 5 years (Group 1995, Parkin, Bray et al. 2005). However, rational administration of these expensive and often toxic treatments requires the accurate identification of (a) patients with localized tumors but with a high risk of relapse, and (b) patients with distant metastases or micrometastases that are refractory to localized therapies (Veronesi, Luini et al. 1993, Fisher, Anderson et al. 1995). Moreover, it is important to find objective methods to monitor the response of patients to different treatments.

At present estrogen and progesterone receptors (ER and PgR, respectively) and uPA/PAI-1 proteases measured in the tumor tissue are clinical parameters that are commonly used to predict the course of the disease and/or to select patients who may benefit from endocrine therapy in breast cancer. ER and PgR are proteins that belong to the family of nuclear receptors: a portion of these proteins binds to specific sections of DNA in the cell, another part of the molecule binds instead to specific molecules such as the steroid hormones estrogen and progesterone. Urokinase-type plasminogen activator (uPA), its receptor (uPAR) and inhibitor (PAI-1), are enzymes which play a key role in the degradation and remodeling of the extracellular matrix of tissues.

Many therapeutic agents that are used in the current clinical practice target the estrogen-ER complex (McGuire 1980, Gion, Mione et al. 1985, Gion, Dittadi et al. 1991, Dittadi, Meo et al. 1997, Bardou, Arpino et al. 2003, Duffy 2005). Overall, the presence of ER and PgR in the primary tumor indicates a better prognosis, which is due in part to the biology of the disease and in part to the benefits that can be obtained in response to therapy. The demonstration that the simultaneous determination of ER and PgR significantly improves the separation between responsive and non-responsive breast cancers with respect to endocrine therapy, has led many laboratories to enter the dosage of both receptors in the routine analyses of breast biopsies (Piffanelli, Giovannini et al. 1986).

On the other hand, a high level of uPA/PAI-1 is generally associated with an aggressive disease and patients who show such levels of these proteins are characterized by reduced survival. uPA and PAI -1 were often found to be the most effective parameters, after the number of positive lymph nodes, in predicting survival. In particular, the surgically treated patients who have high levels of these two biomarkers had doubled the risk of relapsing or dying from this disease (Sweep, Geurts-Moespot et al. 1998, Paradiso, Volpe et al. 2002).

Numerous tumor markers have been proposed for breast cancer. These include mucins of the MUC-1 family (e.g. the CA 15.3, BR 27.29, MCA, CA 549 glycoproteins), the carcinoembryonic antigen (CEA), oncoproteins (eg HER-2/c-erbB-2) and cytokeratins (e.g. tissue polypeptide antigen, TPA; specific tissue polypeptide antigen, TPS. The TPA test is a broad spectrum test that measures cytokeratins 8, 18 and 19. TPS and CYFRA 21-1 are instead more specific and measure cytokeratin 18 and cytokeratin 19 respectively) (van Dalen 1996). The combination of one MUC-1 marker with the CEA marker is often recommended in breast cancer patients. Tumor marker measuring could provide additional guidance beyond the pathological stage of the disease: high levels of CA 15.3 and/or CEA in patients with apparently localized disease could suggest the presence of metastases (Jager, Eibner et al. 2000, Ebeling, Stieber et al. 2002). Elevated levels of CEA (e.g.> 20 ng / ml) and CA 15.3 (e.g.> 50 U / ml) have been found in 40-50% and 50-70% respectively of patients with distant metastases (Molina, Zanon et al. 1995, Tomlinson, Whyman et al. 1995, Molina, Jo et al. 1996, Jager, Eibner et al. 2000). The simultaneous use of these two markers improves the diagnosis of metastases (especially to the bones and liver) in a fraction of patients with breast carcinoma. However, most of these markers have poor specificity and sensitivity. This prevents the use of such markers for screening and diagnosis, especially in the early stages of the disease (Oncology 1996, Molina, Jo et al. 1998). In fact, low levels of tumor markers in patients with presumed breast cancer cannot exclude the presence of the tumor pathology. On the other end, monitoring the tumor marker levels after an initial treatment in patients with breast cancer can be useful in the detection of possible metastasis or relapse.

The increasing knowledge of the mechanisms that regulate apoptosis and tumor growth can give a fundamental contribution to the discovery of new circulating tumor biomarkers (Hanahan and Weinberg 2000). The Authors of the present invention have shown that the monomeric membrane glycoprotein Trop-2 is expressed at high levels in the vast majority of human carcinomas (Trerotola, Cantanelli et al. 2013, Ambrogi, Fornili et al. 2014), including breast, colorectal, endometrium, kidney, ovary, stomach, lung and prostate tumors. In a similar way the homologous Trop-1 molecule is expressed at hgh levels in aggressive, rapidly growing breast cancers, in lung cancers (Piyathilake, Frost et al. 2000) and in the majority of colon tumors (Girardet, Vacca et al. 1986). Consistent with tumor overexpression, Trop-1 is able to stimulate proliferation and metastization of tumor cells (Wurfel, Rosel et al. 1999, Munz, Kieu et al. 2004, Zanna, Trerotola et al. 2007). The Authors of the present invention have shown that Trop-2 plays a similar role. Trop-2 overexpression stimulates tumor cell proliferation both *in vitro* and *in vivo* (Trerotola, Cantanelli et al. 2013, Ambrogi, Fornili et al. 2014) and it is important for tumor development (Alberti Saverio "Anti-Trop-2 monoclonal antibodies and uses thereof in the treatment and diagnosis of tumors" - PCT/WO2010089782). The Authors cloned the human *TROP2* gene (Stein, Basu et al. 1993, Fornaro, Dell'Arciprete et al. 1995) and the gene for the murine *Trop-2* (Szala, Froehlich et al. 1990, De Leij, Helrich et al. 1994, El Sewedy, Fornaro et al. 1998), and characterized these sequences, thus identifying conserved aminoacidic residues that can play a key role in signal transduction activation. In particular the Authors discovered that the cytoplasmic tail of both the human and the murine Trop-2 contains a conserved serine that can be phosphorilated by PKC (Basu, Goldenberg et al. 1995), and a phosphatidylinositol-binding consensus sequence (Alberti 1998, El Sewedy, Fornaro et al. 1998, Alberti 1999, Ciccarelli, Acciarito et al. 2000). Moreover the Authors of the present invention showed that Trop-2 stimulates an increase in cytoplasmic calcium levels following cross-linking (Ripani, Sacchetti et al. 1998). Cytoplasmic Ca²⁺ plays an important role in the regulation of the physiological responses of mammalian cells (Tsien and Tsien 1990, Meldolesi, Clementi et al. 1991). In particular cytoplasmic Ca²⁺ is involved in the cell cycle regulation of actively proliferating cells and especially tumor cells (Dixon, Bowler et al. 1997). Therefore Trop-2 is a key signal transduction molecule that drives fundamental signalling pathways for cell growth (Guerra, Trerotola et al. 2013, Trerotola, Cantanelli et al. 2013) (Alberti Saverio, Guerra Emanuela "Use of Trop-2 as a predictive marker of response to anti-tumor therapy based on inhibitors of CD9, AKT, and molecules of the tetraspanin signalling network.- PCT/WO2013171777). Reliable non-invasive measurement of tumor burden could provide an opportunity for the non-invasive monitoring of the development of the disease and the response to therapy in cancer patients. This would allow early detection and the ability to promptly begin systemic therapy, treating more aggressively the cancer patients who really need it. This would optimized the benefit/cost ratio, where the costs are both the toxic effects associated with more aggressive therapies, and the actual cost of the treatments to be administered.

Traditional methods for cancer diagnosis suffer from serious limitations. For example, imaging diagnostic techniques (including mammography, ultrasound, MRI, PET) are widely used for mass screening (O'Flynn, Wilson et al. 2010, Gutwein, Ang et al. 2011, Williams, Yao et al. 2011), but have limited accessibility and questionable cost/benefit ratio. Furthermore, the sensitivity of these methods may be insufficient. For example, in the case of breast cancer mammography and ultrasound are not able to detect tumors that are smaller than 0.5 cm of diameter. In the case of aggressive tumors, it is possible that a tumor of that size has already spread to other parts of the body, making the necessary therapies extremely difficult and risky for the patient.

Molecules that induce tumor proliferation, such as EGFR and HER-2/neu membrane receptors (Sandri, Johansson et al. 2007), may be released in soluble form in the circulating blood as a result of proteolytic cleavage. Molecules that are released in soluble form in the bloodstream are often subjected to enzymatic attack (e.g. proteolytic degradation) which causes rapid elimination of such molecules. Only in the case in which these substances have instead a half-life in the bloodstream that is long enough to allow them to remain in the circulation, it may be possible in principle to use them as circulating tumor markers. Such markers are potentially able to reveal the presence of the disease at the earliest stages of its development, particularly in the most aggressive tumors. However the tumors markers that have been used so far do not have sufficient sensitivity and specificity to be used effectively (Gion and Daidone 2004).

Similarly to Trop-1 (Schon, Schön et al. 1993), Trop-2 can be proteolytically processed (Stoyanova, Goldstein et al. 2012), with the release of the cytoplasmic tail, which is then translocated into the nucleus, where it acts as a signal inducer. The patent application WO2011109440 describes how in peripheral blood it is possible to detect the presence of Trop-2 in vesicles. Also Chen and colleagues (Chen, Lai et al. 2012) have identified the Trop-2 protein in particulate fractions that were isolated from urinary fluid from patients with bladder cancer. Such urinary fluid collects the proteic material that is released locally by the tumor into the bladder lumen. When the Trop-2 levels were measured again on unfractionated urine by ELISA using the polyclonal anti-Trop-2 AF650 commercial antibody (R&D Systems, Inc.), about a quarter of the bladder cancer patients had Trop-2 values below the the cut-off. Western blot techniques could provide better performance the ELISA assays, since they enable at the same time the detection of the signal produced by immunobinding to Trop-2 and the assessment of the molecular weight that corresponds to the measured signals. This double specificity means that spurious signals can be identified and the signal/noise ratio can be improved.

The antibody that is used to detect Trop-2 plays a key role in determining the sensitivity and specificity of the assay. Polyclonal antibodies are a collection of immunoglobulin molecules that target different epitopes on a molecule, thus recognizing multiple sites on an antigen. Tipically they have high sensitivity (different antibody molecules bind to one antigen molecule) and tolerate changes in the antigen such as sequence polymorphisms or denaturation, and are therefore preferred for Western blotting applications with SDS-PAGE gel electrophoresis. However, polyclonal antibodies also include non-specific and/or low affinity antibodies, are variable from batch to batch, and are necessarily produced in animals. Monoclonal antibodies, on the contrary, are produced from a single B cell against a single epitope, and are therefore homogeneous and consistently reproducible. Moreover, the use of animals is limited to the first phase of antibody generation (preparation of splenocytes from immunized animal, usually mice or rats), while the subsequent antibody production can be performed indefinitely *in vitro,* even on a large scale. Despite these potential advantages, monoclonal antibodies can vary greatly in their affinity towards the target molecule; they may display low sensitivity, since they recognize a single epitope and bind the target molecule with a 1:1 ratio; they may fail to recognize epitope variants. Hence obtaining a suitable monoclonal antibody requires a great effort, with no guarantee of success, and it is often considered to be too demanding for detection purposes.

The use of labelled secondary antibodies to detect antigen-antibody complexes , while enhancing the sensitivity of the assay, is also a source of spurious signal. In fact secondary antibodies can show low level of cross-recognition between different species. This could result in false positives when applied to a IgG-rich sample (such as serum) and this would be especially apparent in conditions of maximal sensitivity of the assay.

### Summary of the invention

The Authors of the present invention have investigated whether the Trop-2 that is expressed by tumor cells is released in soluble form in the circulating blood and if this Trop-2 has sufficient stability and resistance to proteolytic degradation to remain in the bloodstream in measurable quantities. The Authors also investigated whether the amount of Trop-2 in unfractionated serum from cancer patients is a circulating biomarker that associates with tumor burden and/or progression and with response to therapy. To do this the Authors have applied Western blot analysis with anti-Trop-2 antibodies to samples of unfractioned serum from patients with breast carcinoma or adenoma, patients with colorectal carcinoma, and healthy donors. For the breast cancer patients, tumor tissue samples were also analyzed in parallel. Western blot analyses were performed using a polyclonal anti-Trop-2 antibody that had been generated by the Authors by rabbit immunization with a recombinant Trop-2 protein purified from bacteria that had been engineered with *TROP2* expression vectors. A commercial goat anti-Trop 2 polyclonal antibody (AF650 from R&D Systems, Inc.) and anti-Trop-2 monoclonal antibodies were also used. The Authors also measured hormone receptor and uPA / PAI-1 protease markers in the breast cancer patient group and compared them with the new Trop-2 biomarker to assess its potential and added value.

As a result, the Authors of the present invention have shown for the first time that the Trop-2 protein measured in the unfractionated serum of patients with different types of cancer can be used as a new tumor biomarker that is non-invasive and able to reveal the presence of tumor cells with specificity of up to 100%. The Authors have determined that Trop-2 is released and remains in the bloodstream and that the circulating Trop-2 levels vary significantly, with higher levels associated with cancer patients. In addition, the Authors have shown that the circulating Trop-2 levels measured in the unfractionated serum correlate with tumor stage, where the highest levels of circulating Trop-2 are associated with tumors at more advanced pathological stages. The Authors have also developed a novel Western blot assay that can detect Trop-2 in serum samples with high sensitivity, specificity and robustness by means of specific, directly-labelled anti-Trop-2 monoclonal antibodies and samples run under non-reducing conditions.

### Description of the invention

Therefore a specific object of the present invention is a method for the monitoring of the presence of disease in a patient, wherein the disease consists in local or metastatic cancer. This method is characterized in that Trop-2 is measured directly in the serum as obtained from the patient, without the need to perform fractionations, enrichments in specific fractions, or washes, and the Trop-2 levels measured in these conditions allow to monitor the tumor load of this same patient. Hence circulating Trop-2 is a tumor biomarker that is directly usable in the clinic for non-invasive monitoring of local or metastatic tumors. The local or metastatic tumors which can be monitored by using the Trop-2 protein as a biomarker according to the present invention can be non-exclusively selected in the group that consists of breast, colorectal, stomach, pancreas, prostate, cervix, endometrium, ovary, lung, bladder, kidney and thyroid cancers.

Another specific object of the present invention is a method where serum is taken from the same patient at two or more times and the Trop-2 protein that is present in all of the serum samples is measured, said method characterized by the fact that an increase in the levels of the Trop -2 protein in the last sample of at least 10% compared to the previous sample indicates local or metastasic tumor progression, while decreased levels of the Trop-2 protein in the last sample of at least 10% compared to the previous sample indicates local or metastatic tumor regression.

A further object of the present invention is a method for evaluating the effectiveness of cancer therapy in a tumor patient, wherein serum samples are taken from the patient before the start of the antitumor therapy and during or after the antitumor therapy and the levels of the Trop-2 protein in all of the serum samples are measured, and characterized by the fact that a decrease of at least 10% of the Trop-2 protein levels in serum taken from the patient during or after treatment, compared to the levels of the Trop -2 protein in serum taken from the same patient before therapy indicates therapeutic efficacy.

It is also an object of the present invention a method to evaluate anticancer therapy effectiveness in a patient with local or metastatic cancer, characterized by the fact that Trop-2 in the patient serum is used as biomarker. The anti-cancer therapies whose effectiveness can be monitored using the Trop-2 protein as a biomarker according to the present invention can be chosen in a non-exclusive manner in the group consisting of surgery, radiation, chemotherapeutic drugs, alkylating agents, topoisomerase inhibitors, antimetabolites, anticancer antibiotics, mitotic inhibitors, corticosteroids, differentiating agents, hormonal therapy, kinase inhibitors, anti-cancer antibodies, proteasome inhibitors, either alone or combined with each other, for the treatment of local or metastatic tumors non-exclusively selected in the group that consists of breast, colorectal, stomach, pancreas, prostate, cervix, endometrium, ovary, lung, bladder, kidney and thyroid cancers.

It is also an object of the present invention a method for the measurement of the Trop-2 biomarker in the serum, where a molecule or a compound capable of specifically binding the human Trop-2 protein is used, together with a method to quantify this bond. The molecule or compound able to specifically bind the human Trop-2 protein can be an antibody or an antibody fragment, or a chemical derivatization thereof, such as conjugation with enzymes, fluorophore labelling, or biotin labelling, as non-exclusive examples, and corresponding ligands, such as avidin or anti-biotin antibodies or biotin-binding peptides as non-exclusive examples. In one embodiment of the present invention in-house raised anti-Trop-2 polyclonal antibodies are used to detect the presence of Trop-2 in the serum.

In a preferred embodiment the Trop-2-binding antibody or antibody fragment, derivative or conjugate is or derives from the 2EF monoclonal antibody produced by the hybridoma deposited at the Advanced Biotechnology Center (ABC) of Genoa (Italy) with the number PD 08021, or the 2G10 monoclonal antibody produced by the hybridoma deposited at the Genoa ABC with the number PD 08020, or the 4F6 monoclonal antibody produced by the hybridoma deposited at the Genoa ABC with the number PD 08019. These antibodies are taught in the patent application WO 2010/089782. In a further preferred embodiment the Trop-2-binding antibody or antibody fragment, derivative or conjugate is or derives from a chimeric or humanized antibody that recognises and binds the same epitopes or epitope as the 2EF or 2G10 or 4F6 monoclonal antibodies as above, as taught in the patent number WO 2016/087651. Recognition of the same epitope by different antibodies can be demonstrated by binding competition experiments using full-length antigens. The method for the detection/quantification of the antibody binding to the human Trop-2 protein can use as non-exclusive example substances in the group consisting of fluorescent molecules, molecules that are labelled with radioactive isotopes, enzymes that produce spectrophotometry-detectable substances.

In an embodiment of the present invention these can be conjugated to a secondary antibody (two-step detection).

In a preferred embodiment of the invention the labelling molecules is directly linked to the anti-Trop-2 (primary) antibody as described above, for one-step detection.

In one embodiment of the invention the method for the detection of Trop-2 in whole serum uses Western blotting technique. In a preferred embodiment the serum samples are analyzed by Western blotting in denaturing but non-reducing conditions, i.e.with SDS but in the absence of reducing agents such as merchaptoethanol or DTT. These conditions preserve the cysteine disulphide bridges of the Trop-2 globular domain (aa 31-145 in P099758.3) which was found by the Authors to contain the epitope that is recognized by the 2EF antibody (Alberti Saverio: "Anti-Trop-2 monoclonal antibodies and uses thereof in the treatment and diagnosis of tumors" PCT/WO 2010/089782).

It is further described a method for measuring the levels of the Trop-2 biomarker in non-fractionated serum by means of competitive immunobinding with competitors that are labelled with fluorophores or radioactive isotopes or with other techniques. In one of these techniques known amounts of recombinant Trop-2 fused with a fluorescent molecule are added to the serum sample to be analyzed. The fluorescent Trop-2 competes with the circulating Trop-2 for binding to specific antibodies, in solution or immobilized on solid matrices. In this way enzymatic reactions (such as the ones used in ELISA or chemiluminescence assays) are avoided, as well as common sources of inaccuracy of the conventional techniques of immunofluorescence labelling and detection e.g. binding to target proteins of a stochastically variable number of fluorophore molecules, or quenching of intramolecular fluorescence, which causes artifactual reduction in the fluorescent signal. Furthermore the recombinant fluorescent Trop-2 competitor can be engineered for specific mutations/post-translational modifications and allow the measurement of tumor-specific Trop-2 isoforms, for example, glycosylation variants, by means of corresponding antibodies (Ambrogi, Fornili et al. 2014).

It is also an embodiment of the present invention a method for measuring the levels of the Trop-2 biomarker in unfractionated serum that is based on immunoenzymometric assays (IEMA), or affinity chromatography. Further described is a kit for the detection and quantification of the serum levels of the Trop-2 biomarker by specific immunobinding, said kit comprising or consisting of an antibody or an antibody fragment, including a scFv, a Fv fragment, a Fab, fragment, a F(ab)2 fragment, a bifunctional hybrid antibody, a multimeric antibody, or an immunoconjugate containing, by way of non-exclusive example, fluorophores, enzymes, or radioactive isotopes. Such kit can also include as a non-exclusive example protein standards, and/or detection means, and/or a manual of instruction. In a preferred embodiment the antibody, or antibody fragment or derivative or conjugate is the 2EF or the 2G10 or the 4F6 monoclonal antibody or corresponding chimeric or humanized antibodies or antibodies that bind to the same Trop-2 epitopes that are recognized by 2EF or 2G10 or 4F6. In a preferred embodiment the anti-Trop-2 antibody, or antibody fragment or derivative or conjugate is directly conjugated to horseradish peroxidase (hrp).

### Industrial applicability

The present invention finds application in the clinical field for the monitoring of local or metastatic cancer, where the Trop-2 protein in the serum of tumor patients constitutes a non-invasive biomarker. As a non-exclusive example Trop-2 can be quantified in cancer patient sera that have been obtained from a venous blood sampling with the techniques that are known in the art. The serum may be fresh or frozen, and can be used as such or diluted, as non-exclusive example, in PBS, to optimize the signal / noise ratio, according to the examples that are described below. The quantification of Trop-2 can be comparative, for example by comparing the quantity of the Trop-2 present in the serum of a patient with cancer with the quantity of the Trop-2 present in a mixture of sera from cancer-free individuals, or absolute, comparing the quantity of the Trop-2 present in the serum of a patient with cancer with known amounts of purified or recombinant Trop-2 protein, in accordance with the use of reference standard curves known in the art.

Tumor evolution over time and during or after therapy can be followed for example by taking multiple blood samples at different times and quantifying the Trop-2 protein in the sera taken at different times either by comparison or with reference to an internal standard. The initial sample can act as a reference for assessing changes in Trop-2 levels. For this purpose, sera may be stored according to techniques known in the art, for example in a -20 ° C or -80 ° C freezer to allow multiple and subsequent analysis.

Fluorescent and non-fluorescent recombinant Trop-2 can be produced through the recombinant DNA techniques that are known in the art. A nucleic acid molecule that codes for Trop-2 fused to a fluorophore may be generated for example by gene synthesis or by PCR amplification. The nucleic acid molecule can be cloned into an expression vector, with the recombinant DNA techniques that are known in the art. A vector for the expression of recombinant fluorescent Trop-2 can be inserted into host cell lines for the production of the corresponding protein, for example bacteria, yeasts, insect cells, mammalian cells, by transformation, transfection, infection. These methods are known in the art and corresponding kits are commercially available. Recombinant fluorescent Trop-2 can be stored within the host cell or secreted, and can be purified from cell lysates or from the culture medium according to the techniques that are known in the art. Mutant / modified Trop-2 forms can be produced with the recombinant DNA techniques that are known in the art.

Anti Trop-2 antibodies can be produced, purified and labelled with the standard techniques that are known in the art.

The present invention will be now described by way of illustration and example, according, but not limited, to, some of its preferred embodiments, with particular reference to the figures of the enclosed drawings.

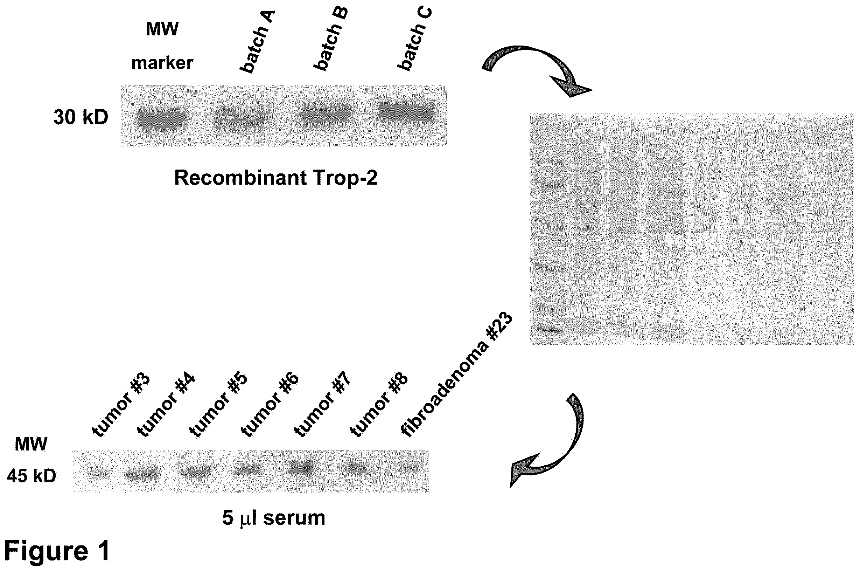

**Figure 1****: Technical approach.** (*top left*) Recombinant human Trop-2 was purified by affinity chromatography on on Ni²⁺-NTA resin and used as an immunogen to obtain anti-Trop-2 antibodies. (*mid*) PAGE electrophoresis of breast cancer patient sera followed by Ponceau red staining; a patient with fibroadenoma is included as a control. (bottom left) Western blot analysis of breast cancer patient sera with anti-Trop-2 antibodies; a patient with fibroadenoma is included as a control.

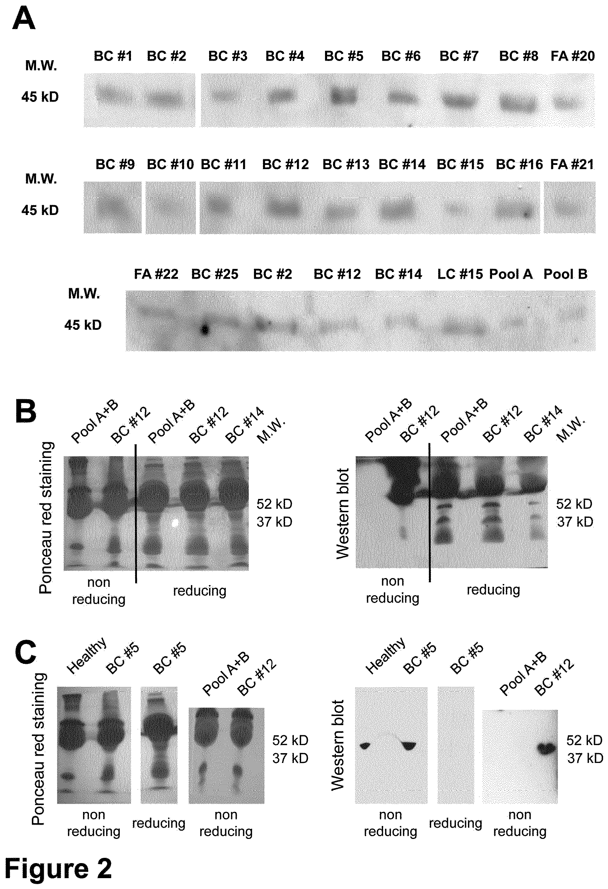

**Figure 2****: Western blot analysis of circulating Trop-2 levels in breast cancer patients.** Circulating Trop-2 was quantified by Western blot in unfractionated sera from patients with breast cancer or fibroadenoma (F.A.). Five µl of unfractionated serum were run in each lane and Trop-2 levels were measured using a rabbit polyclonal antibody generated by the Authors of the present invention; one case of lung cancer was included as histotype control (A). Sera were also assayed in parallel under non reducing and reducing conditions with a commercially available biotinilated anti-Trop-2 polyclonal antibody (cat. AF650, R&D Systems, Inc.) and streptavidin-hrp (B) and with the 2EF monoclonal anti-Trop-2 antibody directly conjugated to hrp (C), revealed by SuperSignal™ West Femto Maximum Sensitivity Substrate. Normal A, B: pools of unfractionated sera from healthy donors: sera from different healthy individuals were combined and run in the respective lanes. (B, C). The Ponceau red staining of the pre-hybridization filters is shown (left), together with the detected hrp signal (right).

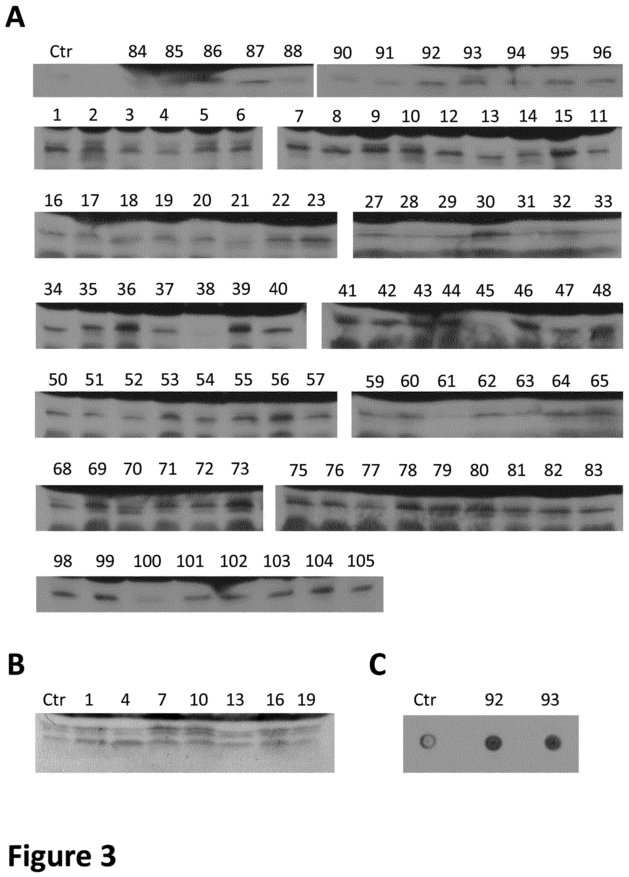

**Figure 3****: Western blot analysis of circulating Trop-2 levels in colorectal cancer patients.** Circulating Trop-2 was quantified in unfractioned sera from 105 colorectal cancer patients at different stages of aggressiveness. Five µl of unfractionated serum were run in each lane, and Trop-2 levels were measured using a rabbit polyclonal antibody generated by the Authors of the present invention (A) or a commercially available goat polyclonal anti-Trop-2 antibody (cat. AF650, R&D Systems, Inc.) (B). "Dot blot" analysis of unfractionated sera from colorectal cancer patients using a monoclonal anti-Trop-2 antibody (C). Ctr : pools of unfractionated sera from healthy donors: sera from different healthy individuals were combined and run in the respective lanes.

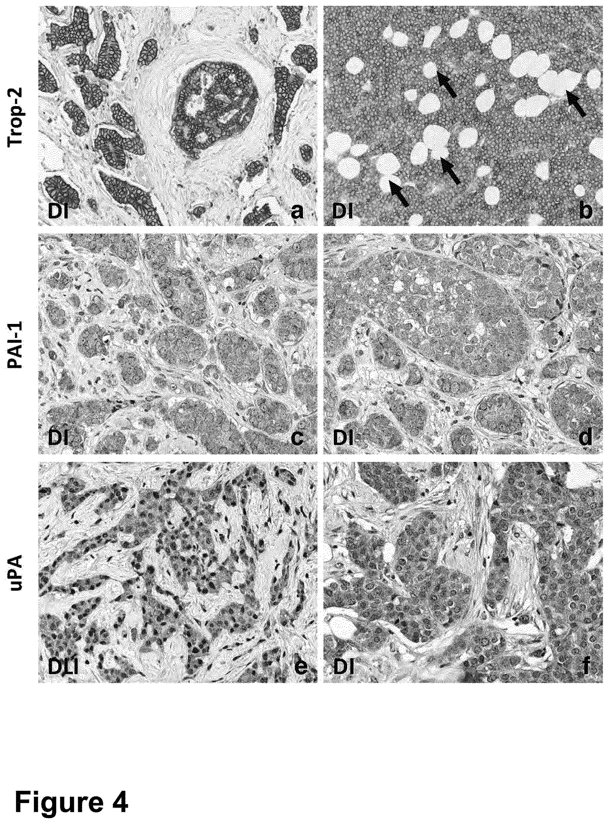

**Figure 4****: IHC analysis of breast cancer pathology determinants.** Trop-2 was quantified by IHC in breast cancer samples together with pathological determinants of tumor progression. (*top*) Trop-2, (*mid*) PAI-1, (*bottom*) uPA; representative examples are shown. DI: ductal infiltrating; DLI: ducto-lobular infiltrating. a, b: 20x; c-f: 40x objectives. Arrows: infiltrated fat tissue.

### EXAMPLE 1 - Feasibility study of the use of circulating Trop-2 as measured in unfractionated serum as a tumor biormarker.

The proof of concept study that shows the feasibility and usefulness of considering soluble Trop-2 in the serum as a tumor biomarker was carried out by recruiting patients and collecting the necessary samples at the Regional Centre for Biochemical Tumor Indicators, Hospital of Venice, and Units of Surgery and Surgical Pathology, San Giacomo Hospital, Castelfranco Veneto (breast cancer) and at the Bari University hospital (colorectal cancer). The Regional Centre is the Italian Reference Center of the EORTC *Receptors and Biomarker Study Group.* In this study 25 patients were recruited who were aged between 20-80 years and had undergone surgery (mastectomy, quadrantectomy, lumpectomy) for primary breast tumor or benign disease (e.g. fibroadenoma, breast reduction, etc..). Written consent was obtained from all the participants to the study, which was approved by the ABO board. For each patient a serum sample and two tissue samples (one from normal tissue and one from the tumor or the benign disease) were obtained. Normal tissues were obtained at least 2 cm away from the lesion. Necrotic parts and fat were removed from all tissue samples. These were then cut in smaller fragments, divided into aliquots and frozen in liquid nitrogen. Seven ml of blood were collected from each patient. Blood samples were left at room temperature for about 2 hours for coagulation to occur. Clotted samples were centrifuged at 1500 g for 20 min, and the serum supernatant was collected. Serum aliquots were then quickly frozen in liquid nitrogen. Tissue and serum aliquots were transported and stored at -80 °C. For each patient a complete medical history was obtained (name, surname, date of birth, social status, habits, menopausal status) as well as clinical and pathological data (histopathological diagnosis, TNM staging, tumor diameter, surgery and/or therapy) (Table 1). Sera from 22 patients with breast cancer, 3 patient undergoing surgery for begnin fibroadenoma and 2 healthy volunteers were analyzed. Sera were collected 1 to 7 days before surgery and were stored at -80°C.

The cohort of colorectal cancer patients was recruited at the University Hospital of Bari. These patients were aged between 30 and 90 years and had undergone surgery with resection of specific colorectal sections (Table 2). Written consent was obtained from all the participants to the study, which was approved by the University Hospital of Bari board. For each patient a serum sample and two tissue samples (one from normal tissue and one from the tumor or the benign disease) were obtained, together with the medical history (name, surname, date of birth, family history) and the main clinical and pathological data (histopathological diagnosis, TNM staging, presence of distant metastases, follow-up duration, current status) (Table 2). The samples were processed as previously described, and stored at -80 ° C. Sera from 105 patients with colorectal cancer at different stages of tumor progression and 3 healthy volunteers (for measurement of the basal levels of serum Trop-2) were analyzed.

### Statistical analysis

Univariate statistical analyses were performed with GraphPad Prism (GraphPad Software Inc., La Jolla, Ca) and XLStat (Addinsoft, Paris, France). Spearman's correlation analysis was performed using MetaboAnalyst 2.0 software (www.metaboanalyst.ca) (Xia, Psychogios et al. 2009, Xia and Wishart 2011, Xia, Mandal et al. 2012). Multivariate statistical analysis and data modeling were performed using MetaboAnalyst 2.0 (www.metaboanalyst.ca) (Xia, Psychogios et al. 2009, Xia and Wishart 2011, Xia, Mandal et al. 2012).and SIMCA 13 (Umetrics, Umea, Sweden) (Eriksson, Antti et al. 2004) software packages.

### Recombinant Trop-2 production

In this study circulating Trop-2 levels were assessed by Western blotting, using a polyclonal anti-Trop-2 antibody that was produced by the Authors. To obtain efficient anti-Trop-2 polyclonal antibodies. recombinant human Trop-2 was produced in bacteria, using pET or pQE (Qiagen, Chatsworth, CA) expression vectors (Fornaro, Dell'Arciprete et al. 1995). Constructs encoding N-ter 6-His tagged Trop-2 extracytoplasmic region (comprised between the leader sequence and the transmembrane region) (Fornaro, Dell'Arciprete et al. 1995) were amplified in *Escherichia coli* XL1-blue (Stratagene, La Jolla, CA), and transformed in the BL21/pLysS strain (Promega, Fitchburg, WI) for protein production. Recombinant Trop-2 synthesis was induced by IPTG 0.2 mM for 5 hours. The recombinant protein was purified by affinity chromatography on Ni²⁺-NTA columns (Qiagen, Chatsworth, CA) (Rodriguez, Talamantez et al. 1998), under denaturing conditions (8 M urea) according to the protcol of the manufacturer. Purity and yield were verified by SDS-PAGE. Recombinant human Trop-2 appeared as an essentially pure single band of approximately 30 kD (Figure 1).

### Production and purification of di anti-Trop-2 polyclonal antibodies

Anti-Trop-2 polyclonal antibodies were obtained by immunizing New Zealand White rabbits (Charles River Laboratories, Wilmington, MA) with purified recombinant human Trop-2, following standard protocols (Zanna, Trerotola et al. 2007). Immune rabbit sera were purified by antigen-affinity chromatography on N-hydroxysuccinimide (NHS) Sepharose (GE Healthcare Bio-Sciences AB, Uppsala, Sweden) conjugated with purified recombinant human Trop-2 according to standard protocols (Sacchetti and Alberti 1999, Sacchetti, Cappetti et al. 2001). Conjugation yields were verified by SDS-PAGE analysis of flow-through solutions. Purified polyclonal anti-Trop-2 antibodies were quantified by SDS-PAGE and Coomassie staining, and titrated by Western blot and flow cytofluorimetric analyses.

### One-step Trop-2 detection with monoclonal antibodies

Purified polyclonal and monoclonal anti-Trop-2 antibodies were conjugated with horseradish peroxidase (hrp) using the hrp conjugation kit ab102890 (Abcam, Cambridge, UK) according to the manufacturer instructions. The hrp-antibody conjugates were titrated by Western blot against purified Trop-2 protein and cell lysates from Trop-2 expressing HCT166 colon cancer cell transfectants. Among the monoclonal antibodies that were tested the 2EF-hrp antibody conjugate displayed the highest sensitivity, which was similar to that of the rabbit anti-Trop-2 polyclonal antibody. The labelled antibodies were then tested on unfractionated serum samples in Western blot assays in non reducing and reducing conditions, revealed by SuperSignal™ West Femto Maximum Sensitivity Substrate; the AF650 biotinilated anti-Trop-2 polyclonal antibody was used for comparison (Figure 2, B - C). Non reducing conditions preserve protein disulphide bridges and were predicted to maintain the conformation of the Trop-2 globular domain that is specifically targeted by the 2EF monoclonal antibody. In these conditions polyclonal anti-Trop-2 antibodies showed a very high level of background signal (Figure 2, B). On the contrary the 2EF-hrp could detect a single Trop-2 band with absolute specificity (Figure 2, C).

### Trop-2 serum levels in breast and colorectal cancer as revealed by Western blot

Western blotting procedures to reveal the presence and the levels of Trop-2 in the serum of breast and colorectal cancer patients were optimized to obtain the highest signal-to-noise ratio, utilizing minimum amounts of serum. Briefly, sera aliquots were centrifuged for 30 min at 4 °C and run on SDS-PAGE After transfer to nitrocellulose filters, filters were pre-hybridized in 5% powdered milk (blocking solution) for 2 hours. Hybridization with rabbit anti-Trop-2 antibodies (produced and purified as described above) was subsequently performed in 5% milk for 2 hours at room temperature. The filter was washed for 20 min and incubated with donkey anti-rabbit secondary antibody conjugated with peroxidase (Calbiochem, La Jolla, CA). After a 20 min wash antibody binding was detected by chemiluminescence (ECL: enhanced chemiluminescence; GE Healthcare Bio-Sciences AB, Uppsala, Sweden). To optimize Trop-2 revealing method different absolute amounts of serum (2.5 µl to 10 µL) were assessed by Western blotting at different dilution in PBS, with different amounts of polyclonal rabbit anti-human Trop-2 antibodies. The best signal-to-noise ratio was obtained using 5 µl of serum that was diluted 1:10 in PBS and hybridizing filters with 10 µg of polyclonal rabbit anti-human Trop-2 diluted in 20 ml of blocking solution. The reproducibility of this procedure was verified on multiple donors in at least two independent experiments. Using these standardized settings, Trop-2 levels in cancer patient serum samples were systematically assessed.

On the basis of Trop-2 serum levels, the samples analyzed were divided into three classes: barely detectable (+/-), low (+), medium (++) and high (+++) levels of expression (Table 3). Trop-2 was found to be present in all normal samples at barely detectable levels. On the contrary all higher-than-baseline Trop-2 serum levels were found in patients with breast cancer (100% specificity). Among breast cancers, 17 out of 22 (77% sensitivity) showed elevated levels versus unaffected individuals (Figure 2, Table3). Of note, benign fibroadenomas, albeit expressing high levels of Trop-2, did not show increased Trop-2 serum levels (Table 3). Trop-2 circulating levels showed rather broad dynamic ranges in breat cancer patients. The highest levels (≥++) were found in a subset of tumor samples (12/22; 54%). This suggested a correlation with other parameters that are linked to tumor development (see below). The 2EF-hrp monoclonal antibody was also used on a subset of samples and confirmed the presence of higher levels of circulating Trop-2 levels in tumor samples with respect to healthy controls.

Analysis of circulating Trop-2 levels in coloractal cancer patient sera was performed by quantifying the intensity of the bands that were obtained by Western blotting with a dedicated software that measured band grey intensities with respect to the background. Sixty-seven out of 105 (63.8%) colorectal tumors showed serum Trop-2 levels that were at least 10% higher that basal levels.

### Immunohistochemistry (IHC)

Breast cancer tissue samples were formalin-fixed, paraffin-embedded and subjected to immunoistochemistry (IHC) staining for Trop-2 using the peroxidase-streptavidin-biotin procedure (Querzoli, Pedriali et al. 2006). Five µm thick tissue sections were laid on negatively charged glass slides, deparaffinized in Histoclear, rehydrated in descending alcohol series and washed with distilled water. Endogenous peroxidase activity was blocked with 3% hydrogen peroxide in methanol for 10 min. Heat-induced epitope retrieval was performed in urea 6 M (pH 7.2) for 10 min. Tissue sections were incubated with goat polyclonal anti-Trop-2 (p)Ab (AF650; R&D Systems, Inc. Minneapolis, MN), at a dilution of 1:25 for 40 min at room temperature, followed by incubation with rabbit anti-goat IgG (Santa Cruz Biotechnology, CA). Finally, the tissue sections were incubated with HRP detection reagent kit (TS-125-HR, LabVision) and 3,3-diaminobenzidine (DAKO), 10 min at room temperature. Nuclei were counterstained with Mayer's hematoxylin for 3 seconds. Slides were dehydrated with ascending alcohol series and mounted. Sections from tumor samples were evaluated for the presence of Trop-2 positive cells (0= no positive cells, 1= 1-9%, 2= 10-49%, 3= 50-79%, 4= 80-100%). Staining intensity was then categorized according to the following scores: 0: no staining, 1: weak, 2: intermediate, 3: intense staining. The percentage and intensity scores were multiplied to obtain an overall immunohistochemical score (IHS), ranging from 0 to 12. Signal "volumes", i.e. percent multiplied by intensity values (without categorization), were also obtained, (Table 2).

### Binding assay for ERα and PgR in fresh/frozen tumor samples

Estrogen (ER) and progesteron (PgR) receptors were measured in fresh or frozed breast cancer biopsies according to the dextran coated radioligand binding assay (LBA) reference method. Briefly, gragments of tumor tissues were cleaned from necrotic areas and fat, cut into smaller pieces and transferred into a Teflon capsule, prechilled in liquid nitrogen, containing a tungsten carbide sphere for frozen tissue fragmentation upon shaking. The pulverized tissue was transferred to prechilled, calibrated polycarbonate tubes and dissolved phosphate buffer at 4 °C. Centrifugation for 1h at 4 °C at 100,000 g allowed to separate the cytosolic supernatant, to be assayed for levels of ERα and PgR. This was done by measuring binding to ³H-estradiol, and displacement by cold ligand. Standard binding curves were generated utilizing six serial dilutions of ³H-estradiol (final concentration: 4 nM to 0.125 nM). Unlabeled estradiol was used for displacement assays at 100-fold higher concentration than the highest concentration of ³H-estradiol. Cold and/or labeled estradiol solutions were added to tumor samples supernatants, and incubated at 4 °C for 19 hours. A suspension of charcoal-dextran (500 µl) was then added to each sample and the mixture was incubated 10 min on ice under shaking. The charcoal-dextran suspension was utilized to trap the estradiol (whether labeled or unlabeled), that did not bind the ER receptors. The samples were centrifuged for 15 min at 4,000 g at 4 °C, and the supernatants, containing the receptors-ligand complexes, were counted by scintillation. As receptor binding can reveal non-specific components, only cold-ligand-dispaceable binding was taken into account. To this end, Scatchard plots were drawn, in which the concentration of labeled ligand present in the samples, was represented as a function of bound and unbound radioactivity. Linear fitting of measurements indicated a correct performance of the assay. Specific binding had dissociation constants of 1x10⁻⁹ to 1x10⁻¹⁰ M.

The total protein content was determined using the Coomassie Protein Assay kit (Pierce), a modification of the Coomassie protein-dye binding colorimetric method. The labeled ligand specifically bound to the sample (Fm/ml) was normalized by the total protein content, and expressed as Fm/mg of total protein. The sensitivity limit for ERα and PgR assays was approximately 5 Fm/ml. Validated cut-off values ≥ 10 Fm/mg of protein were utilized to indicate expression of ERα and PgR.

### uPA and PAI-1 expression profiling

The concentration of uPA and PAI-1 was measured with enzyme-linked immunosorbement assay (ELISA) immunometric procedures, using kits from American Diagnostica Inc. In each assay, aliquots (100 µl) of tissue samples prepared according to the same procedure described for the determination of hormone receptors, were inoculated in 96 wells pre-coated with antibodies specific for uPA or PAI-1. The sample-antibody mixture was incubated for 18-20 hours at 4 °C or on ice. The complex formed by target molecules and antibodies was revealed using biotinylated secondary antibodies for 1h at room temperature (sandwich ELISA). After washing, streptavidin-enzyme were added and incubated for 1 h at room temperature. Substrate was added and the mixture was incubated at room temperature for 20 min for spectrophotometric colorimetry. Absolute amounts were determined by comparison with a calibration curve containing known and scalar pure uPA or PAI-1.

Quality control procedures were routinely implemented, on tests execution and to assess results reliability. The limit of sensitivity of the two assays was found to be 0.6 ng/ml for uPA, 6 ng/ml for PAI-1. Samples with greater than or equal to 3 ng/mg for uPA and 14 ng/mg for PAI-1, were considered positive for the expression of the two proteases.

The amounts of uPA and PAI-1 were expressed as a function of the total protein content of the samples (ng/mg of total protein).

### Circulating Trop-as tumor biomarker

Western blot analysis indicated that measurable levels of Trop-2 are released and retained into the bloodstream of cancer patients. In breat cancer patients Trop-2 was detedtable in all of the sera that were analysed, with 100%. Moreover, 77% of breast cancer patients showed increased levels of circulating Trop-2 with respect to healthy controls (77% sensitivity). Hence correlation between circulatingTrop-2 levels in breast cancer patient serum and pathological and clinical variables related to tumor aggressiveness (pathological stage, tumor volume, metastatic diffusion) was investigated, as well as the serum Trop-2 correlation with experimental parameters such as cytoplasmic versus membrane Trop-2 expression in the tumor tissues (Ambrogi, Fornili et al. 2014).

All the sera with the highest serum Trop-2 levels (+++) were found to be associated to advanced stage tumors (pT3 or pT2N+) (Table 1 e Table 2).

IHC analyses showed an association between serum Trop-2 levels and Trop-2 membrane expression in the tumor cells (breast cancer # 7, 9 e 16). A stronger correlation was observed between the tumor volume, the expression levels of serum Trop-2 and the stage of disease. This correlates with the product of time of tumor persistence in the body and of tumor Trop-2 release over time. Notably, the Trop-2 protein is stable and this determines *in vivo* persistence and as a consequence the high circulating Trop-2 levels associated with tumor burden. Consistent with this, correlation with pathological stage was found in 6 patients with breast cancer (samples # 2, 4, 5, 6, 8, 11) (Table 1 and Table 2). Half of these samples (sera # 2, 5 and 8) have elevated Trop-2 serum levels and these were associated with worse pathological stage (extended tumor burden and lymph node metastases), while the three other samples (serum # 4, 6 and 11) were associated with intermediate amounts of Trop-2 in the bloodstream and were related to a less severe pathological stage. It should be noted that a correlation between Trop-2 serum levels and total levels of expression (in the cytoplasm and in the membrane) of this protein, because Trop-2 may be released into the blood stream in response to events of cell lysis, such as necrosis (due to phenomena of hypoxia) or tumor specific apoptosis. Infact high levels of tissue Trop-2, as for example essentially 100% positive cells in fibroadenomas, are not sufficient per se to cause the release of Trop-2 into the bloodstream.

Circulating Trop-2 levels were additionally correlated with tumor proteolytic balance (uPA/PAI-1; Table 3). At least 10% of the samples (sera # 6 and 7) showed a correlation between proteolytic index (uPA/PAI-1) and circulating Trop-2 levels, suggesting involvement of distinct proteolytic pathways and/or tumor cell lysis.

### Origin of the biological material and informed consent for human biological material

The biological samples that were used come from the San Giacomo hospital/ASL 8 of Castelfranco Veneto (Italy), Unit of Cytodiagnostics and Pathological Anatomy and Histology, and from the University hospital of Bari (Italy). An informed consent was obtained in accordance with current regulations.

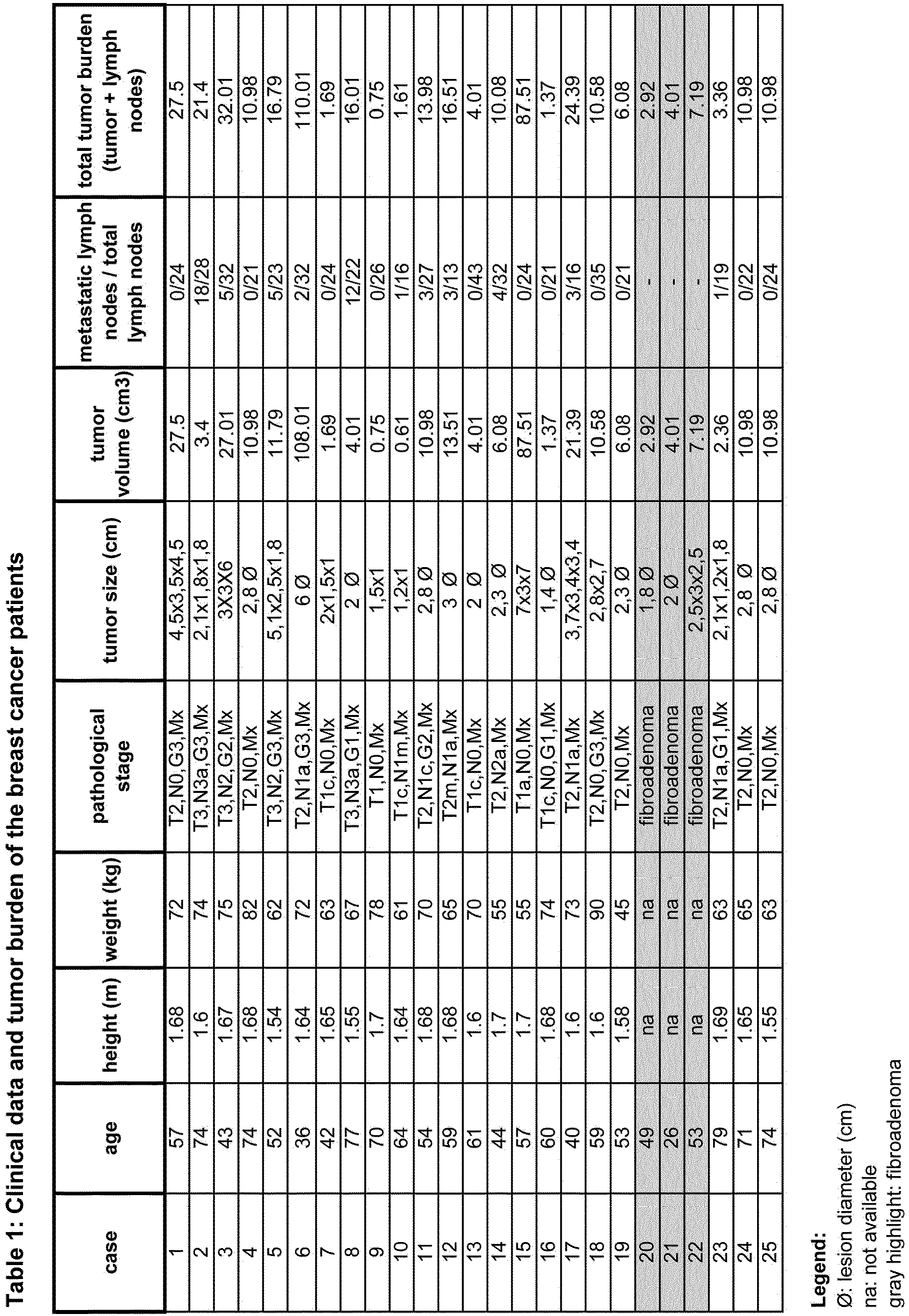

**Table 2: Clinical and follow-up data and Trop-2 serum levels in colorectal cancer patients**

| **case** | **sex** | **age** | **pathological stage** | **distant metastases** | **site of metastasis** | **metastatic lymph nodes / total lymph nodes** | **neoadjuvant RCT** | **Trop-2 serum level increase over baseline** |
|---|---|---|---|---|---|---|---|---|
| **1** | M | 68 | poorly differentiated mucinous adk; B2; T3N0M0 | NO | | 0/13 | NO | 89% |
| **2** | M | 76 | well differentiated adk; T3N1M0 | NO | | 2/13 | NO | 151% |
| **3** | F | 79 | moderately differentiated adk; T3N2M1 | YES | liver | 8/22 | na | 91% |
| **4** | M | 70 | moderately differentiated adk; T2N0M1 | YES | liver | 0/14 | NO | 113% |
| **5** | M | 42 | well differentiated adk; T3N2M0 | NO | | na/34 | na | 138% |
| **6** | M | 78 | well differentiated adk; T2N0M0 | NO | | 0/19 | NO | 98% |
| **7** | M | 39 | poorly differentiated mucinous adk; T4N2M1 | YES | liver | 9/29 | NO | 158% |
| **8** | M | 78 | moderately differentiated adk; T3N0M0 | NO | | 0/15 | NO | 147% |
| **9** | M | 64 | poorly differentiated adk; T3N0M0 | NO | | 0/39 | NO | 98% |
| **10** | M | 77 | well differentiated adk; T1N0M0 | NO | | 0/14 | NO | 120% |
| **11** | M | 61 | moderately differentiated adk; T3N1M0 | NO | | 2/21 | na | 104% |
| **12** | M | 48 | moderately differentiated adk; T3N1MX | na | | 2/14 | NO | 164% |
| **13** | F | 69 | moderately differentiated adk; T3N0M1 | YES | liver | 0/17 | NO | 160% |
| **14** | M | 65 | moderately differentiated adk; T3N0M0 | NO | | na/34 | NO | 162% |
| **15** | F | 84 | moderately differentiated adk; T3N0M0 | NO | | 0/10 | NO | 109% |
| **16** | M | 64 | moderately differentiated adk; T4N0M1 | YES | liver | 0/18 | NO | 196% |
| **17** | M | 63 | poorly differentiated adk; T3N0M0 | NO | | 0/17 | NO | 129% |
| **18** | F | 78 | moderately differentiated adk; T3N0M0 | NO | | 0/15 | NO | 233% |
| **19** | M | 76 | well differentiated adk; T3N1M1 | YES | lung | 2/15 | NO | 231% |
| **20** | F | 68 | moderately differentiated adk; T4N1M0 | NO | | 2/21 | NO | 96% |
| **21** | F | 74 | moderately differentiated adk; T4N0M0 | NO | | 0/42 | NO | 122% |
| **22** | M | 74 | poorly differentiated adk; T4N1M1 | YES | liver | 1/15 | NO | 171% |
| **23** | M | 64 | poorly differentiated adk; T4N2M1 | YES | liver | 7/19 | NO | 200% |
| **24** | M | 74 | moderately differentiated adk; T3N0M1 | YES | liver | 0/28 | na | 284% |
| **25** | M | 74 | poorly differentiated adk; T4N3M1 | YES | liver | 10/16 | NO | 191% |
| **26** | F | 83 | well differentiated adk; T2N0M1 | YES | liver | 0/12 | na | 60% |
| **27** | M | 63 | well differentiated adk; T3N1MX | na | | 1/18 | NO | 264% |
| **28** | F | 75 | moderately differentiated adk; T3N0M1 | YES | lung | 0/21 | NO | 111% |
| **29** | F | 83 | moderately differentiated adk; T2N0M1 | YES | lung | 0/19 | NO | 98% |
| **30** | M | 72 | moderately differentiated adk; T3N1M0 | NO | | 2/16 | NO | 253% |
| **31** | F | 68 | poorly differentiated adk; T3N0M0 | NO | | 0/20 | NO | 136% |
| **32** | M | 55 | moderately differentiated adk; T3N0M0 | NO | | 0/10 | NO | 127% |
| **33** | F | 30 | well differentiated adk; T2N0M0 | NO | | 0/16 | NO | 111% |
| **34** | F | 64 | poorly differentiated adk; T3N0M1 | YES | liver | 1/23 | NO | 131% |
| **35** | M | 76 | moderately differentiated adk; T3N1M0 | NO | | 1/11 | na | 148% |
| **36** | F | 68 | intramucosal adk; TisN0M0 | NO | | 0/16 | NO | 162% |
| **37** | M | 74 | moderately differentiated adk; T3N0M1 | YES | duodenum | 0/15 | NO | 181% |
| **38** | F | 57 | well differentiated adk; T3N0M0 | NO | | 0/16 | NO | 126% |
| **39** | M | 64 | moderately differentiated adk; T3N2M0 | NO | | 4/11 | NO | 221% |
| **40** | M | 82 | poorly differentiated adk; T3N1M0 | NO | | 3/19 | NO | 221% |
| **41** | F | 75 | moderately differentiated adk; T3N1M0 | NO | | 1/12 | YES | 102% |
| **42** | F | 59 | moderately differentiated adk; T3N1M1 | YES | liver | 3/13 | NO | 98% |
| **43** | F | 90 | poorly differentiated adk; T3N0M0 | NO | | 0/17 | NO | 36% |
| **44** | M | 73 | well differentiated adk; T2N0M0 | NO | | 0/10 | NO | 31% |
| **45** | M | 77 | moderately differentiated adk; T4N0M0 | NO | | 0/27 | NO | 76% |
| **46** | M | 83 | poorly differentiated adk; T4N0M0 | NO | | 0/2 | NO | 207% |
| **47** | M | 46 | well differentiated adk; T3N1M0 | NO | | 2/37 | NO | 93% |
| **48** | M | 84 | moderately differentiated adk; T2N0M0 | NO | | 0/na | NO | 140% |
| **49** | F | 76 | poorly differentiated adk; T3N0M1 | YES | liver | 0/41 | NO | 124% |
| **50** | M | 86 | moderately differentiated mucinous adk; T3N1M1 | YES | | 2/18 | NO | 107% |
| **51** | F | 51 | moderately differentiated adk; T3N2M1 | YES | liver | 9/34 | NO | 69% |
| **52** | F | 80 | moderately differentiated adk; T3N1M0 | NO | | 1/23 | NO | 107% |
| **53** | M | 82 | moderately differentiated adk; T3N1M1 | YES | liver | 2/pn | NO | 145% |
| **54** | M | 77 | moderately differentiated adk; T3N0M1 | YES | brain | 0/18 | NO | 140% |
| **55** | F | 75 | moderately differentiated adk; C; T1N1M0 | NO | | 1/17 | NO | 124% |
| **56** | F | 59 | moderately differentiated adk; T3N2MX | na | | 8/20 | na | 176% |
| **57** | M | 68 | poorly differentiated adk; T3N2M0 | NO | | 5/22 | NO | 150% |
| **58** | F | 59 | poorly differentiated adk; T4N1M1 | YES | liver | 2/24 | NO | 133% |
| **59** | M | 72 | moderately differentiated adk; T3N2MX | YES | liver | 7/23 | NO | 157% |
| **60** | F | 72 | moderately differentiated adk; T3N1M0 | NO | | 2/22 | NO | 83% |
| **61** | M | 57 | moderately differentiated adk; T3N2M1 | YES | liver | 5/15 | NO | 60% |
| **62** | F | 71 | moderately differentiated adk; T2N0M0 | NO | | 0/5 | na | 193% |
| **63** | M | 76 | moderately differentiated adk; T1N0M0 | NO | | 0/8 | NO | 114% |
| **64** | F | 55 | moderately differentiated mucinous adk; C; T2N1M0 | NO | | 0/32 | NO | 131% |
| **65** | M | 72 | moderately differentiated adk; T1NXM0 | NO | | pn | NO | 145% |
| **66** | F | 77 | poorly differentiated adk; T4N1M1 | YES | liver | 2/12 | NO | 95% |
| **67** | M | 56 | poorly differentiated adk; T4N2M1 | YES | duodenum-peritoneum | 20/35 | NO | 43% |
| **68** | M | 73 | poorly differentiated adk; TnaNnaM0 | NO | | pn | NO | 79% |
| **69** | M | 78 | moderately differentiated adk; C; T3N1M0 | NO | | 1/32 | NO | 61% |
| **70** | M | 83 | na | na | | na | NO | 69% |
| **71** | F | 50 | moderately differentiated adk; T3N0M0 | NO | | 0/13 | NO | 58% |
| **72** | M | 56 | moderately differentiated adk; TXN0M0 | NO | | 0/14 | na | 85% |
| **73** | M | 74 | well differentiated adk; B2; T3N0M0 | NO | | 0/6 | NO | 107% |
| **74** | F | 70 | na | na | | na | na | 110% |
| **75** | F | 62 | poorly differentiated adk; T4N2M1 | YES | liver | 8/14 | NO | 63% |
| **76** | M | 67 | moderately differentiated adk; T4N1MX | na | | 3/20 | na | 63% |
| **77** | M | 84 | well differentiated adk; T4N2M1 | YES | liver | 8/42 | NO | 52% |
| **78** | F | 88 | poorly differentiated adk; T3N1M0 | NO | | 2/14 | NO | 60% |
| **79** | F | 70 | well differentiated adk; T2N1MX | na | | 1/12 | na | 61% |
| **80** | F | 81 | poorly differentiated adk; T2N0M0 | NO | | 0/32 | NO | 64% |
| **81** | M | 91 | well differentiated adk; T3N1M0 | NO | | 2/13 | na | 75% |
| **82** | M | 55 | moderately differentiated adk; B2; T3N0M0 | NO | | 0/15 | NO | 76% |
| **83** | M | 83 | poorly differentiated adk; T3N1MX | na | | 3/16 | NO | 113% |
| **84** | F | 56 | poorly differentiated adk; C; T3N1M0 | NO | | 1/51 | NO | 150% |
| **85** | F | 83 | moderately differentiated adk; T2N0MX | na | | 0/13 | NO | 89% |
| **86** | M | 72 | well differentiated adk; T3N0M1 | YES | liver | 4/0 | NO | 157% |
| **87** | M | 48 | moderately differentiated adk; T3N1M1 | YES | liver | 2/11 | NO | 193% |
| **88** | M | 69 | well differentiated adk; T3N0M1 | YES | liver | 0/15 | NO | 114% |
| **89** | M | 72 | moderately differentiated adk; T3N2M1 | YES | liver | 5/20 | NO | 118% |
| **90** | F | 72 | moderately differentiated adk; T3N2M0 | NO | | 6/13 | NO | 204% |
| **91** | F | 71 | moderately differentiated adk; T3N0M0 | NO | | 0/45 | NO | 193% |
| **92** | M | 77 | moderately differentiated adk; T3N0M1 | YES | liver | 0/43 | NO | 425% |
| **93** | M | 72 | well differentiated adk; T2N0M0 | NO | | 0/36 | NO | 296% |
| **94** | M | 78 | moderately differentiated adk; B2; T3N0M0 | NO | | 0/14 | NO | 204% |
| **95** | M | 81 | moderately differentiated adk; T3N0M1 | YES | liver | 0/8 | NO | 304% |
| **96** | F | 74 | poorly differentiated adk; T4N2M0 | NO | | 8/15 | NO | 450% |
| **97** | M | 73 | well differentiated adk; T4N0M0 | NO | | 0/17 | NO | 150% |
| **98** | M | 76 | moderately differentiated adk; T2N0M0 | NO | | 0/19 | NO | 300% |
| **99** | M | 65 | moderately differentiated adk; T3N0M0 | NO | | 0/16 | NO | 211% |
| **100** | M | 78 | moderately differentiated adk; C; T3N1M0 | NO | | 1/32 | NO | 121% |
| **101** | F | 59 | well differentiated adk; T3N0M0 | NO | | 0/11 | na | 161% |
| **102** | M | 46 | poorly differentiated adk; T4N2M0 | NO | | 6/27 | NO | 125% |
| **103** | M | 71 | well differentiated adk; T3N0M0 | NO | | 0/32 | na | 221% |
| **104** | M | 60 | well differentiated adk; T3N0M0 | NO | | 0/13 | na | 275% |
| **105** | F | 53 | moderately differentiated adk; T3N1M1 | YES | liver | 3/6 | NO | 211% |

| | | | | | | | | |
|---|---|---|---|---|---|---|---|---|
| **Legend:** na: not available pn: palpable nodules | | | | | | | | |

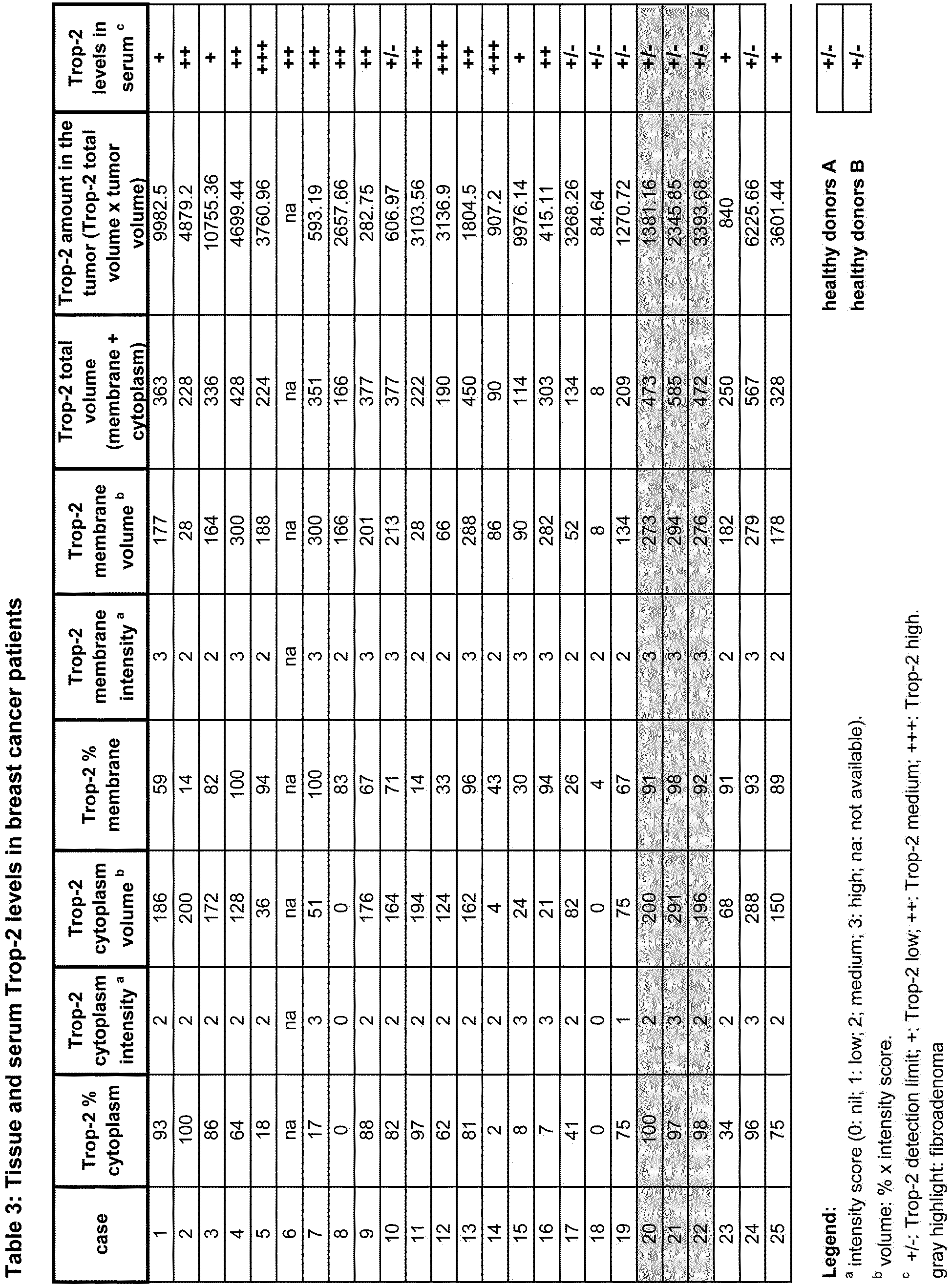

### Bibliography

Alberti, S. (1998). "A phosphoinositide-binding sequence is shared by PH domain target molecules--a model for the binding of PH domains to proteins." Proteins 31(1): 1-9.
Alberti, S. (1999). "HIKE, a candidate protein binding site for PH domains, is a major regulatory region of Gbeta proteins." Proteins 35: 360-363.
Ambrogi, F., M. Fornili, P. Boracchi, M. Trerotola, V. Relli, P. Simeone, R. La Sorda, R. Lattanzio, P. Querzoli, M. Pedriali, M. Piantelli, E. Biganzoli and S. Alberti (2014). "Trop-2 is a determinant of breast cancer survival." PLoS One 9(5): e96993.
Bardou, V. J., G. Arpino, R. M. Elledge, C. K. Osborne and G. M. Clark (2003). "Progesterone receptor status significantly improves outcome prediction over estrogen receptor status alone for adjuvant endocrine therapy in two large breast cancer databases." J Clin Oncol 21(10): 1973-1979.
Basu, A., D. M. Goldenberg and R. Stein (1995). "The epithelial/carcinoma antigen EGP-1, recognized by monoclonal antibody RS7-3G11, is phosphorylated on serine 303." Int J Cancer 62(4): 472-479.
Bonadonna, G., E. Brusamolino, P. Valagussa, A. Rossi, L. Brugnatelli, C. Brambilla, M. De Lena, G. Tancini, E. Bajetta, R. Musumeci and U. Veronesi (1976). "Combination chemotherapy as an adjuvant treatment in operable breast cancer." N Engl J Med 294(8): 405-410.
Bonadonna, G., P. Valagussa, A. Moliterni, M. Zambetti and C. Brambilla (1995). "Adjuvant cyclophosphamide, methotrexate, and fluorouracil in node-positive breast cancer: the results of 20 years of follow-up." N Engl J Med 332(14): 901-906.
Chen, C. L., Y. F. Lai, P. Tang, K. Y. Chien, J. S. Yu, C. H. Tsai, H. W. Chen, C. C. Wu, T. Chung, C. W. Hsu, C. D. Chen, Y. S. Chang, P. L. Chang and Y. T. Chen (2012). "Comparative and targeted proteomic analyses of urinary microparticles from bladder cancer and hernia patients." J Proteome Res 11(12): 5611-5629.
Ciccarelli, F., A. Acciarito and S. Alberti (2000). "Large and diverse numbers of human diseases with HIKE mutations." Hum. Mol. Genet. 9: 1001-1007.
Colditz, G. A., W. C. Willett, D. J. Hunter, M. J. Stampfer, J. E. Manson, C. H. Hennekens and B. A. Rosner (1993). "Family history, age, and risk of breast cancer. Prospective data from the Nurses' Health Study." Jama 270(3): 338-343.
de Koning, H. J., R. Boer, P. G. Warmerdam, P. M. Beemsterboer and P. J. van der Maas (1995). "Quantitative interpretation of age-specific mortality reductions from the Swedish breast cancer-screening trials." J Natl Cancer Inst 87(16): 1217-1223.
De Leij, L., W. Helrich, R. Stein and M. J. Mattes (1994). "SCLC-cluster-2 antibodies detect the pancarcinoma/epithelial glycoprotein EGP-2." Int J Cancer Suppl 8: 60-63.
Dittadi, R., S. Meo, B. Amoroso and M. Gion (1997). "Detection of different estrogen receptor forms in breast cancer cytosol by enzyme immunoassay." Cancer Res 57(6): 1066-1072.
Dixon, C. J., W. B. Bowler, P. Fleetwood, A. F. Ginty, J. A. Gallagher and J. A. Carron (1997). "Extracellular nucleotides stimulate proliferation in MCF-7 breast cancer cells via P2-purinoceptors." Br. J. Cancer 75: 34-39.
Duffy, M. J. (2005). "Predictive markers in breast and other cancers: a review." Clin Chem 51(3): 494-503.
Easton, D. F., D. T. Bishop, D. Ford and G. P. Crockford (1993). "Genetic linkage analysis in familial breast and ovarian cancer: results from 214 families. The Breast Cancer Linkage Consortium." Am J Hum Genet 52(4): 678-701.
Ebeling, F. G., P. Stieber, M. Untch, D. Nagel, G. E. Konecny, U. M. Schmitt, A. Fateh-Moghadam and D. Seidel (2002). "Serum CEA and CA 15-3 as prognostic factors in primary breast cancer." Br J Cancer 86(8): 1217-1222.
Ekbom, A., D. Trichopoulos, H. O. Adami, C. C. Hsieh and S. J. Lan (1992). "Evidence of prenatal influences on breast cancer risk." Lancet 340(8826): 1015-1018.
El Sewedy, T., M. Fornaro and S. Alberti (1998). "Cloning of the murine Trop2 gene: conservation of a PIP2-binding sequence in the cytoplasmic domain of Trop-2." Int J Cancer 75(2): 324-330.
Eriksson, L., H. Antti, J. Gottfries, E. Holmes, E. Johansson, F. Lindgren, I. Long, T. Lundstedt, J. Trygg and S. Wold (2004). "Using chemometrics for navigating in the large data sets of genomics, proteomics, and metabonomics (gpm)." Anal Bioanal Chem 380(3): 419-429.
Fisher, B., S. Anderson, C. K. Redmond, N. Wolmark, D. L. Wickerham and W. M. Cronin (1995). "Reanalysis and results after 12 years of follow-up in a randomized clinical trial comparing total mastectomy with lumpectomy with or without irradiation in the treatment of breast cancer." N Engl J Med 333(22): 1456-1461. Fornaro, M., R. Dell'Arciprete, M. Stella, C. Bucci, M. Nutini, M. G. Capri and S. Alberti (1995). "Cloning of the gene encoding TROP-2, a cell-surface glycoprotein expressed by human carcinomas." Int. J. Cancer 62: 610-618.
Gion, M. and M. G. Daidone (2004). "Circulating biomarkers from tumour bulk to tumour machinery: promises and pitfalls." Eur J Cancer 40(17): 2613-2622.
Gion, M., R. Dittadi, A. E. Leon, G. Bruscagnin, D. Pelizzola, G. Giovannini, M. Giganti, G. Messeri, M. Quercioli, E. Flamini and et al. (1991). "Comparison between single saturating dose ligand binding assay and enzyme immunoassay for low-salt extractable oestrogen and progesterone receptors in breast cancer: a multicentre study." Eur J Cancer 27(8): 996-1002.
Gion, M., R. Mione, R. Dittadi, L. Griggio, G. Munegato, M. Valescchi, O. Del Maschio, S. Fasan and G. Bruscagnin (1985). "Estrogen and progesterone receptors in breast carcinoma and in nonmalignant breast tissue." Tumori 71(5): 477-481.
Girardet, C., A. Vacca, A. Schmidt-Kessen, M. Schreyer, S. Carrel and J. P. Mach (1986). "Immunochemical characterization of two antigens recognized by new monoclonal antibodies against human colon carcinoma." J. Immunol. 136(4): 1497-1503.
Group, E. B. C. T. C. (1995). "Effects of radiotherapy and surgery in early breast cancer. An overview of the randomized trials.." N Engl J Med 333(22): 1444-1455. Guerra, E., M. Trerotola, A. L. Aloisi, R. Tripaldi, G. Vacca, R. La Sorda, R. Lattanzio, M. Piantelli and S. Alberti (2013). "The Trop-2 signalling network in cancer growth." Oncogene 32: 1594-1600.
Gutwein, L. G., D. N. Ang, H. Liu, J. K. Marshall, S. N. Hochwald, E. M. Copeland and S. R. Grobmyer (2011). "Utilization of minimally invasive breast biopsy for the evaluation of suspicious breast lesions." Am J Surg 202(2): 127-132.
Hanahan, D. and R. A. Weinberg (2000). "The hallmarks of cancer." Cell 100(1): 57-70.
Jager, W., K. Eibner, B. Loffler, S. Gleixner and S. Kramer (2000). "Serial CEA and CA 15-3 measurements during follow-up of breast cancer patients." Anticancer Res 20(6D): 5179-5182.
Madigan, M. P., R. G. Ziegler, J. Benichou, C. Byrne and R. N. Hoover (1995). "Proportion of breast cancer cases in the United States explained by well-established risk factors." J Natl Cancer Inst 87(22): 1681-1685.
McGuire, W. L. (1980). "Steroid hormone receptors in breast cancer treatment strategy." Recent Prog Horm Res 36: 135-156.
Meldolesi, J., E. Clementi, C. Fasolato, D. Zacchetti and T. Pozzan (1991). "Ca2+ influx following receptor activation." Trends Pharmacol.Sci. 12: 289-292.
Molina, R., J. Jo, X. Filella, G. Zanon, J. Pahisa, M. Mu noz, B. Farrus, M. L. Latre, C. Escriche, J. Estape and A. M. Ballesta (1998). "c-erbB-2 oncoprotein, CEA, and CA 15.3 in patients with breast cancer: prognostic value." Breast Cancer Res Treat 51(2): 109-119.
Molina, R., J. Jo, G. Zanon, X. Filella, B. Farrus, M. Munoz, M. L. Latre, J. Pahisa, M. Velasco, P. Fernandez, J. Estape and A. M. Ballesta (1996). "Utility of C-erbB-2 in tissue and in serum in the early diagnosis of recurrence in breast cancer patients: comparison with carcinoembryonic antigen and CA 15.3." Br J Cancer 74(7): 1126-1131.
Molina, R., G. Zanon, X. Filella, F. Moreno, J. Jo, M. Daniels, M. L. Latre, N. Gimenez, J. Pahisa, M. Velasco and et al. (1995). "Use of serial carcinoembryonic antigen and CA 15.3 assays in detecting relapses in breast cancer patients." Breast Cancer Res Treat 36(1): 41-48.
Morrow, M. (1994). "Identification and management of the woman at increased risk for breast cancer development." Breast Cancer Res Treat 31(1): 53-60.
Munz, M., C. Kieu, B. Mack, B. Schmitt, R. Zeidler and O. Gires (2004). "The carcinoma-associated antigen EpCAM upregulates c-myc and induces cell proliferation." Oncogene.
Newcomb, P. A., B. E. Storer, M. P. Longnecker, R. Mittendorf, E. R. Greenberg and W. C. Willett (1996). "Pregnancy termination in relation to risk of breast cancer." Jama 275(4): 283-287.
O'Flynn, E. A., A. R. Wilson and M. J. Michell (2010). "Image-guided breast biopsy: state-of-the-art." Clin Radiol 65(4): 259-270.
Oncology, A. S. o. C. (1996). "Clinical practice guidelines for the use of tumor markers in breast and colorectal cancer. Adopted on May 17, 1996." J Clin Oncol 14(10): 2843-2877.
Paradiso, A., S. Volpe, A. Iacobacci, E. Marubini, P. Verderio, A. Costa, M. G. Daidone, A. Marchetti, M. Mottolese, D. Amadori, F. De Paola, L. Saragoni, L. Medri, I. Nenci, P. Querzoli, M. Gion, R. Dittadi, M. Plebani, C. Orlando, G. Bevilacqua and R. Silvestrini (2002). "Quality control for biomarker determination in oncology: the experience of the Italian Network for Quality Assessment of Tumor Biomarkers (INQAT)." Int J Biol Markers 17(3): 201-214.
Parker, S. L., T. Tong, S. Bolden and P. A. Wingo (1996). "Cancer statistics, 1996." CA Cancer J Clin 46(1): 5-27.
Parkin, D. M., F. Bray, J. Ferlay and P. Pisani (2005). "Global cancer statistics, 2002." CA Cancer J Clin 55(2): 74-108.
Piffanelli, A., G. Giovannini, D. Pelizzola and M. De Bortoli (1986). "Estrogen and progesterone measurement and its quality control in breast cancer: a reappraisal." Int J Biol Markers 1(1): 15-28.
Piyathilake, C. J., A. R. Frost, H. Weiss, U. Manne, D. C. Heimburger and W. E. Grizzle (2000). "The expression of Ep-CAM (17-1A) in squamous cell cancers of the lung." Hum. Pathol. 31(4): 482-487.
Querzoli, P., M. Pedriali, R. Rinaldi, A. R. Lombardi, E. Biganzoli, P. Boracchi, S. Ferretti, C. Frasson, C. Zanella, S. Ghisellini, F. Ambrogi, L. Antolini, M. Piantelli, S. Iacobelli, E. Marubini, S. Alberti and I. Nenci (2006). "Axillary lymph node nanometastases are prognostic factors for disease-free survival and metastatic relapse in breast cancer patients." Clin. Cancer Res. 12(22): 6696-6701.
Ripani, E., A. Sacchetti, D. Corda and S. Alberti (1998). "The human Trop-2 is a tumor-associated calcium signal transducer." Int. J. Cancer 76: 671-676.
Rodriguez, K., J. Talamantez, W. Huang, S. H. Reed, Z. Wang, L. Chen, W. J. Feaver, E. C. Friedberg and A. E. Tomkinson (1998). "Affinity purification and partial characterization of a yeast multiprotein complex for nucleotide excision repair using histidine-tagged Rad14 protein." J Biol Chem 273(51): 34180-34189. Sacchetti, A. and S. Alberti (1999). "Protein tags enhance GFP folding in eukaryotic cells." Nat.Biotechnol. 17: 1046.
Sacchetti, A., V. Cappetti, P. Marra, R. Dell'Arciprete, T. El-Sewedy, C. Crescenzi and S. Alberti (2001). "Green Fluorescent Protein variants fold differentially in prokaryotic and eukaryotic cells." J. Cell. Biochem. 36: 117-128.
Sandri, M. T., H. A. Johansson, L. Zorzino, M. Salvatici, R. Passerini, P. Maisonneuve, A. Rocca, G. Peruzzotti and M. Colleoni (2007). "Serum EGFR and serum HER-2/neu are useful predictive and prognostic markers in metastatic breast cancer patients treated with metronomic chemotherapy." Cancer 110: 509-517.
Schön, M. P., M. Schön, M. J. Mattes, R. Stein, L. Weber, S. Alberti and C. E. Klein (1993). "Biochemical and immunological characterization of the human carcinoma-associated antigen MH 99/KS 1/4." Int. J. Cancer 55: 988-995.
Siegel, R. L., K. D. Miller and A. Jemal (2015). "Cancer statistics, 2015." CA Cancer J Clin 65(1): 5-29.
Stein, R., A. Basu, S. Chen, L. B. Shih and D. M. Goldenberg (1993). "Specificity and properties of mab RS7-3G11 and the antigen defined by this pancarcinoma monoclonal antibody." Int. J. Cancer 55: 938-946.
Stoyanova, T., A. S. Goldstein, H. Cai, J. M. Drake, J. Huang and O. N. Witte (2012). "Regulated proteolysis of Trop2 drives epithelial hyperplasia and stem cell self-renewal via beta-catenin signaling." Genes Dev 26(20): 2271-2285.
Sweep, C. G., J. Geurts-Moespot, N. Grebenschikov, J. H. de Witte, J. J. Heuvel, M. Schmitt, M. J. Duffy, F. Janicke, M. D. Kramer, J. A. Foekens, N. Brunner, G. Brugal, A. N. Pedersen and T. J. Benraad (1998). "External quality assessment of trans-European multicentre antigen determinations (enzyme-linked immunosorbent assay) of urokinase-type plasminogen activator (uPA) and its type 1 inhibitor (PAI-1) in human breast cancer tissue extracts." Br J Cancer 78(11): 1434-1441.
Szala, S., M. Froehlich, M. Scollon, Y. Kasai, Z. Steplewski, H. Koprowski and A. J. Linnenbach (1990). "Molecular cloning of cDNA for the carcinoma-associated antigen GA733-2." Proc. Natl. Acad. Sci. USA 87: 3542-3546.
Tabar, L., C. J. Fagerberg, A. Gad, L. Baldetorp, L. H. Holmberg, O. Grontoft, U. Ljungquist, B. Lundstrom, J. C. Manson, G. Eklund and et al. (1985). "Reduction in mortality from breast cancer after mass screening with mammography. Randomised trial from the Breast Cancer Screening Working Group of the Swedish National Board of Health and Welfare." Lancet 1(8433): 829-832.
Tomlinson, I. P., A. Whyman, J. A. Barrett and J. K. Kremer (1995). "Tumour marker CA15-3: possible uses in the routine management of breast cancer." Eur J Cancer 31A(6): 899-902.
Trerotola, M., P. Cantanelli, E. Guerra, R. Tripaldi, A. L. Aloisi, V. Bonasera, R. Lattanzio, R. de Lange, U. H. Weidle, M. Piantelli and S. Alberti (2013). "Up-regulation of Trop-2 quantitatively stimulates human cancer growth." Oncogene 32 222-233.
Tsien, R. W. and R. Y. Tsien (1990). "Calcium channels, stores, and oscillations." Annu. Rev. Cell Biol. 6: 715-760.
van Dalen, A. (1996). "Significance of cytokeratin markers TPA, TPA (cyk), TPS and CYFRA 21.1 in metastatic disease." Anticancer Res 16(4B): 2345-2349.
Veronesi, U., A. Luini, M. Del Vecchio, M. Greco, V. Galimberti, M. Merson, F. Rilke, V. Sacchini, R. Saccozzi, T. Savio and et al. (1993). "Radiotherapy after breast-preserving surgery in women with localized cancer of the breast." N Engl J Med 328(22): 1587-1591.
Williams, R. T., K. Yao, A. K. Stewart, D. J. Winchester, M. Turk, A. Gorchow, N. Jaskowiak and D. P. Winchester (2011). "Needle versus excisional biopsy for noninvasive and invasive breast cancer: report from the National Cancer Data Base, 2003-2008." Ann Surg Oncol 18(13): 3802-3810.
Wurfel, J., M. Rosel, S. Seiter, C. Claas, M. Herlevsen, R. Weth and M. Zoller (1999). "Metastasis-association of the rat ortholog of the human epithelial glycoprotein antigen EGP314." Oncogene 18: 2323-2334.
Xia, J., R. Mandal, I. V. Sinelnikov, D. Broadhurst and D. S. Wishart (2012). "MetaboAnalyst 2.0--a comprehensive server for metabolomic data analysis." Nucleic Acids Res.
Xia, J., N. Psychogios, N. Young and D. S. Wishart (2009). "MetaboAnalyst: a web server for metabolomic data analysis and interpretation." Nucleic Acids Res 37(Web Server issue): W652-660.
Xia, J. and D. S. Wishart (2011). "Web-based inference of biological patterns, functions and pathways from metabolomic data using MetaboAnalyst." Nat Protoc 6(6): 743-760.
Zanna, P., M. Trerotola, G. Vacca, V. Bonasera, B. Palombo, E. Guerra, C. Rossi, R. Lattanzio, M. Piantelli and S. Alberti (2007). "Trop-1 is a novel cell growth stimulatory molecule that marks early stages of tumor progression." Cancer 110(2): 452-464.

## Claims

1. Method for non-invasive *in vitro* monitoring of the presence of tumor disease in a patient wherein the Trop-2 protein present in the unfractionated serum sample from the patient is used as a circulating tumor biomarker, the presence of tumor disease being indicated by levels of circulating Trop-2 protein at least 10% higher than the levels of circulating Trop-2 protein in corresponding healthy individuals.

2. Method according to claim 1, wherein the tumor disease consists in local or metastatic tumors selected from the group consisting of cancers of the breast, colon-rectum, stomach, pancreas, prostate, uterine cervix, endometrium, ovary, lung, bladder, kidney and thyroid.

3. Method according to claim 1, wherein the serum is taken at two or more different times from the same patient and the Trop-2 protein present in all the serum samples is measured, **characterized by** the fact that an increase in the levels of the Trop-2 protein in the later sample of at least 10%, compared to the earlier sample, is used to indicate tumor progression.

4. Method according to claim 1, wherein the serum is taken at two or more different times from the same patient and the Trop-2 protein present in all the serum samples is measured, **characterized by** the fact that a decrease in the levels of the Trop-2 protein in the later sample of at least 10%, compared to the earlier sample, is used to indicate tumor regression.

5. Method to *in vitro* assess the effectiveness of anticancer therapy in a tumor patient, where in serum samples obtained from the patient before the beginning of anticancer therapy and during or after anticancer therapy the levels of the Trop-2 protein present in all the serum samples are measured, and **characterized by** the fact that a decrease of at least 10% of the levels of the Trop-2 protein present in the serum from the patient during or after the therapy, compared to the levels of the Trop-2 protein in the serum from the same patient before the beginning of the therapy, indicates therapeutic efficacy.

6. Method according to claim 5, wherein the anticancer therapy is selected from the group consisting of surgery, radiotherapy, chemotherapy drugs, alkylating agents, topoisomerase inhibitors, antimetabolites, antitumor antibiotics, mitotic inhibitors, corticosteroids, differentiating agents, hormonal therapy, kinase inhibitors, antitumor antibodies, proteasome inhibitors, alone or in combination, for the treatment of local or metastatic tumors according to claim 2.

## Patentansprüche

1. Verfahren zur nicht-invasiven *in-vitro-* Kontrolle des Vorhandensein von Tumorerkrankungen bei einem Patienten, wobei das in der nichtfraktionierten Serumprobe des Patienten vorhandene Trop-2-Protein als Biomarker für einen zirkulierenden Tumor verwendet wird, wobei das Vorhandensein von Tumorerkrankungen durch Niveaus des zirkulierenden Trop-2-Proteins angezeigt wird, und ist mindestens 10% höher als die Zirkulationsniveaus des Trop-2-Proteins bei entsprechenden gesunden Menschen.

2. Verfahren nach Anspruch 1, wobei die Tumorerkrankung aus lokalen oder metastasierenden Tumoren besteht, die aus der Gruppe bestehend aus Krebserkrankungen in der Brust, Kolon-Rektum, Magen, Pankreas, Prostata, Gebärmutterhals, Endometrium, Eierstöcke, Lunge, Blase, Nieren und Schilddrüse, ausgewählt sind.

3. Verfahren nach Anspruch 1, **dadurch gekennzeichnet, dass** das Serum zu zwei oder mehr verschiedenen Zeitpunkten vom gleichen Patienten genommen wird und das in allen Serumproben vorhandene Trop-2-Protein gemessen wird, **dadurch gekennzeichnet, dass** eine Zunahme von mindestens 10% in der Trop-2-Proteingehalte in der nächsten Probe im Vergleich zur vorherigen Probe verwendet wird, um das Fortschreiten des Tumors anzuzeigen.

4. Verfahren nach Anspruch 1, wobei das Serum von demselben Patienten zu zwei oder mehr unterschiedlichen Zeitpunkten genommen wird und das in allen Serumproben vorhandene Trop-2-Protein gemessen wird, **dadurch gekennzeichnet, dass** eine Abnahme von mindestens 10% in den Trop-2-Proteingehalte in der nächsten Probe im Vergleich zur vorherigen Probe verwendet wird, um eine Tumorregression anzuzeigen.

5. Verfahren zur *in-Vitro* Bestimmung der Wirksamkeit einer Krebstherapie bei einem Krebspatienten, wobei in den vom Patienten vor Beginn der Krebstherapie und während oder nach der Krebstherapie entnommenen Serumproben die Niveaus des Trop-Proteins, die in allen Serumproben vorhanden sind gemessen werden, **dadurch gekennzeichnet, dass** eine Abnahme von mindestens 10% der im Serum des Patienten vorhandenen Trop-2-Proteinniveaus während oder nach der Therapie im Vergleich zu den Niveaus des Trop-2-Proteins im Serum von derselben Patienten, vor Beginn der Therapie, die therapeutische Wirksamkeit anzeigt.

6. Verfahren nach Anspruch 5, wobei die Antikrebstherapie ausgewählt ist aus der Gruppe bestehend aus Chirurgie, Strahlentherapie, Chemotherapeutika, Alkylierungsmitteln, Topoisomerase-Inhibitoren, Antimetaboliten, Antitumorantibiotika, Mitose-Inhibitoren, Kortikosteroiden, differenzierenden Mitteln, Hormontherapie, Kinasehemmern, Antitumor-Antikörper, Proteasom-Inhibitoren, allein oder in Kombination, zur Behandlung von lokalen oder metastasierenden Tumoren nach Anspruch 2.

## Revendications

1. Procédé de contrôle *in vitro* non invasive de la présence de maladies tumorales chez un patient, dans laquelle la protéine Trop-2 présente dans l'échantillon de sérum non fractionné du patient est utilisée comme marqueur biologique d'une tumeur en circulation, la présence de maladies tumorales étant indiquée par les niveaux de circulation de la protéine Trop-2 supérieure d'au moins 10% aux niveaux de circulation de la protéine Trop-2 chez les personnes en bonne santé correspondantes.

2. Procédé selon la revendication 1, dans lequel la maladie tumorale est constituée de tumeurs locales ou métastatiques, choisies dans le groupe constitué par les cancers du sein, du côlon-rectum, de l' estomac, du pancréas, de la prostate, du col utérin, de l'endomètre, des ovaires, des poumons, de la vessie, des reins et de la thyroïde.

3. Procédé selon la revendication 1, **caractérisé en ce que** du même patient le sérum est prélève à deux moments différents ou plus et que l'on mesure la protéine Trop-2 présente dans tous les échantillons de sérum, **caractérisé en ce qu'**une augmentation d'au moins 10% des niveaux de la protéine Trop-2 dans l'échantillon suivant, comparés à l'échantillon précédent, sont utilisés pour indiquer la progression tumorale.

4. Procédé selon la revendication 1, dans lequel le sérum est prélevé à deux moments différents ou plus du même patient, et la protéine Trop-2 présente dans tous les échantillons de sérum est mesurée, **caractérisée en ce qu'**une diminution d'au moins 10% des niveaux de protéine Trop-2 dans l'échantillon suivant par rapport à l'échantillon précédent, est utilisé pour indiquer une régression tumorale.

5. Procédé de détermination *in vitro de* l'efficacité du traitement anticancéreux chez un patient cancéreux, dans lequel les niveaux de sérum obtenus du patient avant le début du traitement anticancéreux et pendant ou après le traitement anticancéreux permettent de mesurer les niveaux de la protéine Trop 2 présents dans tous les échantillons de sérum, et **caractérisé en ce qu'**une diminution d'au moins 10% des niveaux de protéine Trop-2 présents dans le sérum du patient, pendant ou après le traitement, comparée aux niveaux de protéine Trop-2 dans le sérum de même patient, avant de commencer le traitement, indique son efficacité thérapeutique.

6. Procédé selon la revendication 5, dans lequel la thérapie anticancéreuse est choisie dans le groupe comprenant la chirurgie, la radiothérapie, des médicaments chimio-thérapeutiques, des agents alkylants, des inhibiteurs de la topoisomérase, des antimétabolites, des antibiotiques antitumoraux, des inhibiteurs de la mitose, des corticostéroïdes, des agents de différentiation, une hormonothérapie, des inhibiteurs de la kinase, des anticorps anti-tumoraux, des inhibiteurs du protéasome, seuls ou en combinaison, pour le traitement de tumeurs locales ou métastatiques selon la revendication 2.
